# EUROPEAN PATENT APPLICATION

(11) **EP 3 981 448 A1**
(43) Date of publication of application: **13.04.2022**
(21) Application number: 20210934.4
(22) Date of filing: 01.12.2020
(51) Int. Cl.: A61M 5/162, A61M 25/02, A61M 5/158

(54) **DRUG INFUSION SET**

(30) Priority: 08.10.2020 KR 20200130550
(71) Applicant: Sooil Development Co., Ltd., Gyenggi-do (KR)
(72) Inventor: CHOI, Soo-Bong, Seoul (KR)
(74) Representative: Becker, Eberhard

(57) **Abstract**

A drug infusion set is provided, in which a catheter fixing unit and a drug infusion unit are mounted/unmounted, the catheter fixing unit includes a body member, which includes a guide unit, in which a receiving space is formed in a downward direction from an upper surface; a drug injection unit, in which an accommodation space is formed in a horizontal direction from the other side to communicate with the guide unit, and into which the drug infusion unit is inserted; and a catheter insertion unit, which extends in a downward direction of the guide unit; a guided insertion member, which is provided with a guide needle, inserted into the guide unit; first and second sealing members, which are inserted into the accommodation space of the guide unit and the drug injection unit; and a catheter, which is inserted into the catheter insertion unit.

## Description

### BACKGROUND

### Technical Field

The present disclosure relates to a drug infusion set in which a drug is injected into the human body through a catheter by removing an injection needle after inserting it into the human body so as to inject a drug to a patient.

### Description of Related Art

In general, vaccinations (*e.g.*, vaccines), insulin for diabetics, anticoagulants (*e.g.*, heparin), and cardiac stimulants are administered by subcutaneous injection. The area for injection is generally the brachii of the upper arm. In the case of insulin, which is frequently injected, if the injection is continuously made only in one area, it is easy to cause induration. Therefore, it is common to sequentially use the areas of the lower abdomen, thighs, buttocks, *etc.* for injections. Due to the nature of insulin, self-injection is easy, and injections are frequently made in the abdomen in which self- injection is easy and has a wide area.

Conventionally known "injection set" of International Publication No. WO 2007/092210 discloses a constitution, in which the needle and the cannula are inserted through the patient's skin and then removed from the base member in a state where the base member is attached to the patient's skin. Therefore, there was a hassle in that an introducer cap and an infusion cap attached to the base member should be provided separately. Additionally, it discloses a method to assemble by compressing to cause the barbed feet to the rim of the base member, in which the infusion set is configured to be disengaged from the rim of the base member by applying a force while gripping the release grips of the introducer cap, and thus there was a problem in that the assembly and disengagement were difficult.

In order to solve such a problem, the "insulin infusion set" disclosed in Korean Patent No. 10-1802110 provides a structure in which a catheter fixing member and a tube connecting member can be easily separated and assembled to each other. Conventionally, Teflon material was mainly used for a catheter so that it can be inserted into the patient's body and move flexibly. However, due to the characteristics of the Teflon material, when bonding using an adhesive, there was a problem in that the adhesion was not achieved and it either slipped or the Teflon was removed. Due to such a problem, a structure in which a connection member for separate fixing, a funnel-type insertion unit, and a catheter are coupled was provided in the insulin infusion set without using an adhesive so as to fix the Teflon material, and a partition wall was integrally formed along the periphery of the catheter so that the catheter can be inserted and fixed in the body.

At this time, the body was injection-molded to form a partition wall in a lower part of the body so that a catheter can be inserted into the body and fixed therein. Teflon, which is used as a material for a catheter, is expensive among fluorine resins, and has resulted in higher manufacturing and purchasing costs, thereby resulting in higher sales costs. In addition, in order to fix the catheter made of Teflon, the number of parts was increased, which resulted in an increase of time to prepare and assemble parts, and the lengthy procedure resulted in an increase of working time.

Additionally, in order to inject insulin into the body from the outside through the insertion hole of an injection needle, there was required a sophisticated work to form a circumferential groove and an injection groove on the outer peripheral surface of a connecting member, and there were difficulties in the precisely manufacturing fine parts and assembling so that the circumferential grooves and injection grooves can be placed in the correct positions. In addition, conventional insulin infusion sets had such a structure where the sealing member is formed to face a funnel-shaped slope of the connecting member and is disposed to be in close contact with the inside of the connecting member, and there was a problem in that insulin infusion sets are not properly sealed between the circumferential groove in the connecting member along which a drug is transported and the junction where the injection groove is combined with the body, and thus, there was a problem in that the inside of the body could not be completely sealed.

Meanwhile, in order to completely remove air from the tube and the injection needle before applying the injection needle to a patient, insulin is taken out by operating a pump and air is discharged through the air hole of the injection needle to the tip of the injection needle, and there was a difficulty in precisely positioning the air hole of the injection needle between the connecting member and the funnel-shaped insertion unit. In addition, since the inside of the body was not completely sealed, and thus, the air in the tube and the needle could not be completely removed through the air hole of the injection needle.

### PRIOR ART DOCUMENT

### PATENT DOCUMENT

International Publication No. WO2007/092210
Korean Patent No. 10-1802110

### SUMMARY

To solve the above problems, an object of the present disclosure is to provide a drug infusion set, in which a catheter is made of polyurethane and fixed by an adhesive and the configuration is simplified by removing unnecessary components, and which has a simple structure such that the flow path of the drug that is formed to flow into the body from the outside is integrally molded inside a body member. The problems are not limited to the technical problems described above, and other technical problems may be derived from the description herein below.

A drug infusion set according to an embodiment of the present disclosure relates to one, in which a catheter fixing unit and a drug infusion unit are mounted/unmounted, wherein, in the catheter fixing unit, a body member, which comprises: a guide unit, in which a receiving space of a pre-set shape is formed in a vertical downward direction from an upper surface, a drug injection unit, in which an accommodation space of a pre-set shape is formed in a horizontal direction from the other side to communicate with the guide unit, and into which the drug infusion unit is inserted; and a catheter insertion unit, which extends in a downward direction of the guide unit to form an adhesive space that extends to the outside of a lower surface; a guided insertion member, which is provided with a guide needle, which is inserted into the guide unit, disposed to be exposed to the outside through the catheter insertion unit in a state where an air discharge hole is formed at the point where the guide unit is communicated with the drug injection unit, and being pierced through the first sealing member; a first sealing member and a second sealing member, which are inserted into the accommodation space of the guide unit and the drug injection unit so as to seal the inside thereof; a catheter, which is inserted into the catheter insertion unit and is formed such that the guide needle is allowed to be separated while surrounding the lower outer circumferential surface of the guide needle; and a flow path, in which the catheter consists of polyurethane, and the adhesive , which is injected from the adhesive space of the catheter insertion unit, is cured by ultraviolet rays to fix the catheter, and the drug is moved inside the body member, are integrally molded.

Additionally, the drug infusion set is characterized in that the body member comprises an air discharge unit, where a discharge space smaller than the accommodation space of the guide unit and the drug injection unit is formed to communicate, and is disposed at a point orthogonal to the central axis of the guide unit and the drug injection unit, and the air discharge hole of the guided insertion member is disposed in the other direction of the air discharge unit so as to discharge the internal air of the drug infusion unit.

Additionally, the drug infusion set is characterized in that the body member comprises: a fusion protrusion unit, which is formed on the top of the guide unit and on the side of the drug injection unit, and is fused to the upper surface of the first and second sealing members as a projection with a pre-set thickness protrudes along the outer circumferential surface; and a fusion groove, which is formed at a set depth along the outer circumferential surface of the fusion protrusion unit on the upper surface, wherein in the fusion protrusion unit, the fusion groove guides the movement of a thermal fusion device so that the fusion protrusion unit is fused to the inside by the thermal welding device and is fused to a surface exposed to the outside of the first and second sealing members so as to fix the first and second sealing members.

Additionally, the drug infusion set is characterized in that the guided insertion member comprises: a guide needle cap, which is coupled to the top of the guide needle to couple and separate the guide needle from the body member; and a protection cap, which is coupled to the bottom of the body member to be separated from the outer circumferential surface of the catheter, and is mounted and removed so as to protect the catheter and the guide needle from the outside.

Additionally, the drug infusion set is characterized in that the body member comprises: an insertion guide groove, which is symmetrically formed so that a groove with a pre-set length is opened from the front to the rear from the other side to one side; and a locking protrusion, which is symmetrically formed at the front and rear ends of the insertion guide groove, consists of a right-angled triangle such that a right-angled surface is disposed on one side and a slope is placed on the other side; and wherein the drug infusion unit comprises: an injection needle, in which one side is inserted into the drug injection unit and through which a drug is injected in a state where the injection needle is penetrated through the second sealing member; a drug injection member, which comprises: a fixed wing unit, in which one side is inserted into the insertion guide groove, which is formed to be spaced apart to both sides of the injection needle and has a slope where the outer surface is inclined downward to one side thereof; a modified wing unit, in which one side is inserted into the inside of the locking protrusion to be fixed, and is formed to be spaced apart to both sides of the fixed wing unit and is bent outward; and an elasticity guide groove, which is formed at the connecting point on the other side of the fixed wing unit and the modified wing unit and is formed to extend outwardly; and a drug injection tube, in which the injection needle is connected to the other end, which is provided through the drug injection member, and which supplies a drug to the injection needle by a pump.

### BRIEF DESCRIPTION OF THE DRAWINGS

FIG. 1 is a cross-sectional view of a drug infusion set according to an embodiment of the present disclosure.
FIG. 2 is a cross-sectional view of a body member according to an embodiment of the present disclosure.
FIG. 3 is a cross-sectional view of a catheter fixing unit according to an embodiment of the present disclosure.
FIG. 4 is a front view showing a state before a catheter fixing unit and a drug infusion unit of the present disclosure are coupled together.
FIG. 5 is a plan view showing a state before a catheter fixing unit and a drug infusion unit of the present disclosure are coupled together.

### DESCRIPTION OF THE EMBODIMENTS

Hereinafter, with reference to the accompanying drawings, embodiments of the present disclosure will be described in detail so that one of ordinary skill in the art to which the present disclosure belongs can easily implement it. The present disclosure can be implemented in many different forms and is not limited to the structure or method described herein.

An embodiment of the present disclosure relate to a drug infusion set, in which a catheter is made of polyurethane and fixed by an adhesive and the configuration is simplified by removing unnecessary components, and which has a simple structure such that the flow path of the drug that is formed to flow into the body from the outside is integrally molded inside a body member.

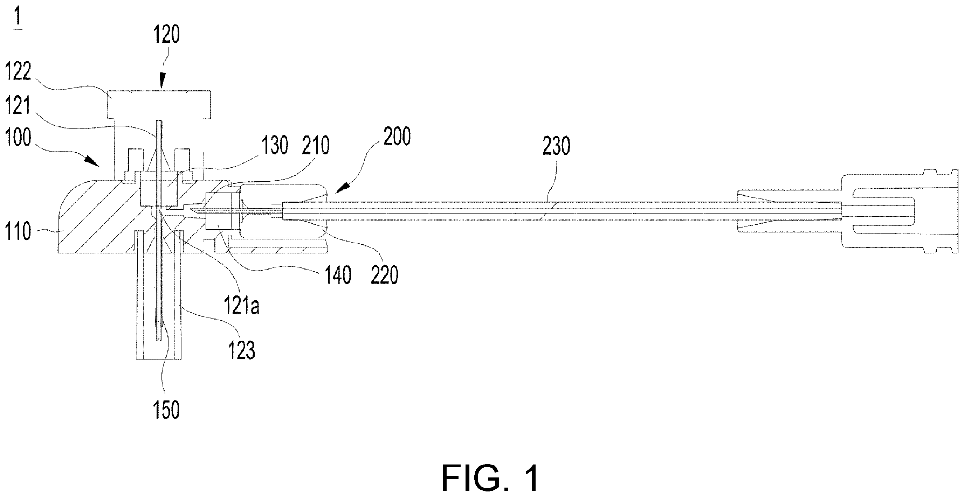

In addition, with respect to the vertical, horizontal directions of the drug infusion set of the present embodiment, based on the front of the drug infusion set shown in FIG. 1, embodiments of the present disclosure will be described by defining the left direction as "one side" and the right direction as "the other side", the ground direction as "downward direction" and the opposite direction as "upward direction". This is for the purpose of explaining the present disclosure so that the present disclosure can be clearly understood, and it goes without saying that each direction can be defined differently depending on where the standard is placed.

It should be noted that the drug infusion set of according to an embodiment of the present disclosure can be mainly used in an administration device for administering insulin to a patient in need of diabetes treatment, but is not limited thereto and can be applied to various devices for administering drugs.

FIG. 1 is a cross-sectional view of a drug infusion set according to an embodiment of the present disclosure.

Referring to FIG. 1, the drug infusion set 1 of the present disclosure is a device used for administering drugs to patients, in which a catheter is made of polyurethane and it is fixed by an adhesive that is cured by ultraviolet rays thus being assembled into a simple structure and a simplified constitution, so that the catheter fixing unit 100 and the drug infusion unit 200 can be mounted/unmounted.

In addition, the drug infusion set 1 consists of a simple structure so that the catheter fixing unit 100 and the drug infusion unit 200 can be mounted/unmounted, and thus, it is easy for anyone to use by removing the guide needle 121 and injecting the drug into the body through the catheter 150 in a state where a catheter 150 and a guide needle 121 of the catheter fixing unit 100 are inserted into the human body, and drugs can be administered periodically without multiple injections into the body.

As shown in FIG. 1, the drug infusion set 1 has a structure in which the drug infusion unit 200 is inserted into the other side of the catheter fixing unit 100 and coupled therein, and the catheter 150 is inserted into the patient's body by a guided insertion member inserted through the upper part of the catheter fixing unit 100, and smoothly supplies the drug injected from the drug infusion unit 200 into the body.

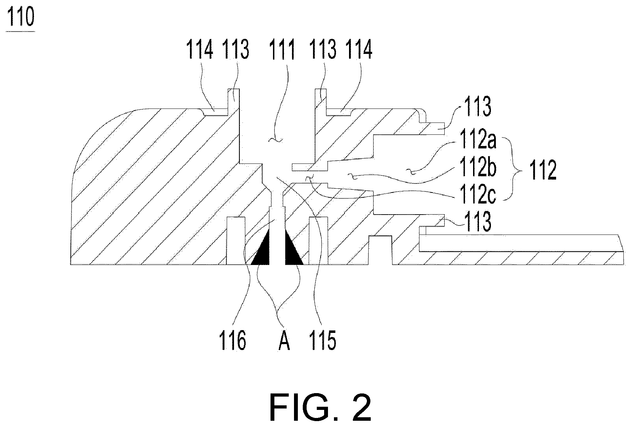

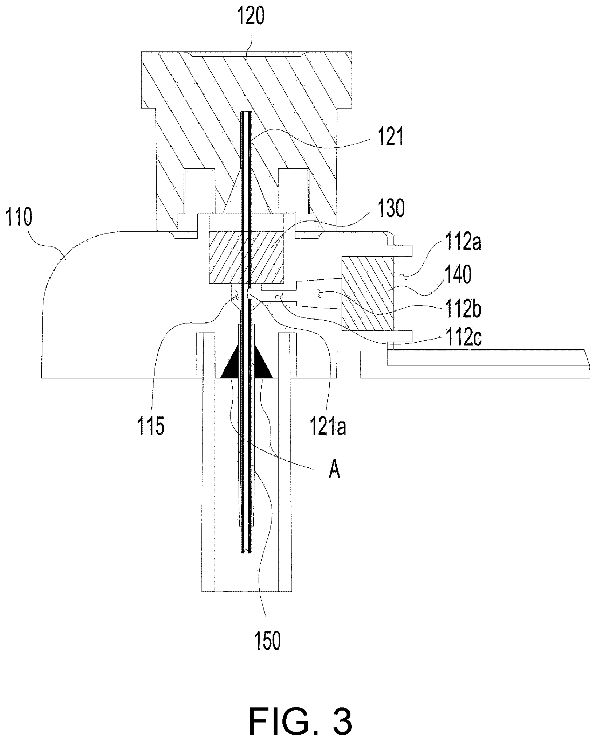

FIG. 2 is a cross-sectional view of a body member according to an embodiment of the present disclosure, and FIG. 3 is a cross-sectional view of a catheter fixing unit according to an embodiment of the present disclosure.

Referring to FIGS. 2 and 3, the catheter fixing unit 100 is formed so that the catheter 150 can be simply fixed by an adhesive A, and the flow path through which the drug, which is injected into the inside from the outside, is injected into the body is formed integrally. Such a catheter fixing unit 100 may include a constitution consisting of a body member 110, a guided insertion member 120, a first sealing member 130, a second sealing member 140, and a catheter 150.

The body member 110 is a constitution in which the flow path through which the drug transports is formed inside and is molded in an integral form so that the drug can be transported to the catheter 150 side, and since the body member 110 is integrally molded, elaborate work is not required. Such a body member 110 may include a constitution consisting of a guide unit 111, a drug injection unit 112, a fusion protrusion unit 113, a fusion groove 114, an air discharge unit 115, a catheter insertion unit 116, an insertion guide groove 117, and a locking protrusion 118.

The guide unit 111 is one which provides a space so that the first sealing member 130 and the guide needle 121 can be inserted, and an accommodation space is formed vertically downward in the center of the upper surface of the body member 110 and it is provided in a state where the upper part is opened. Such a guide unit 111 may be formed in a cylindrical shape, and may be formed to correspond to the first sealing member 130 so that the first sealing member 130 can be inserted in a state being in close contact with the inner wall.

The drug injection unit 112 is one which provides a space to insert a second sealing member 140 and an injection needle 210. An accommodation space of a pre-set shape is formed in the horizontal direction in the center of the other side of the body member 110, and is provided in a state where the other side is opened. Such a drug injection unit 112 may have a plurality of cylinders stepped inward from the other side to one side, and may have a constitution consisting of a sealing member insertion groove 112a, an injection needle location groove 112b, and a drug introduction groove 112c.

The sealing member insertion groove 112a is formed to correspond to the second sealing member 140 on the other side, and it is inserted in a state where the second sealing member 140 is in close contact with the inner wall. Additionally, the injection needle location groove 112b is formed to extend from the sealing member insertion groove 112a to one side thereof, and consists of a diameter smaller than the diameter of the sealing member insertion groove 112a and is stepped inside, and positions the end of an injection needle 210. Additionally, the drug introduction groove 112c is formed to extend from the injection needle location groove 112b to one side thereof, and consists of a diameter smaller than the diameter of the injection needle location groove 112b and is stepped inside, thereby improving the introduction rate of drugs.

The fusion protrusion unit 113 is a constitution which fixes the first and second sealing members 130 and 140 to prevent them being departed to the outside, and is formed at the upper end of the guide unit 111 and a side end of a drug injection unit 112. Additionally, the fusion protrusion unit 113 is formed with a pre-set thickness to protrude along the outer circumferential surface, and fused to the external exposed surfaces of the first and second sealing members 130 and 140 to thereby fuse the first and second sealing members 130 and 140.

The fusion groove 114 guides the movement of a thermal fusion device so that the fusion protrusion unit 113 is fused to the inside by the thermal fusion device, and provides an accommodation space so that the fusion protrusion unit 113 does not protrude outward after it is fused. Such a fusion groove 114 is preferably formed to a pre-set depth along the outer peripheral surface of the fusion protrusion unit 113 and guides the position so that the thermal fusion device can be inserted to the correct position and is thereby provided to apply heat and pressure to the surface of the fusion protrusion unit 113.

The air discharge unit 115 provides a passage for connecting the guide unit 111 and the drug injection unit 112, and is provided to discharge the air within the drug infusion unit 200 to the outside or to inject a drug into the body. Such an air discharge unit 115 is formed into a space smaller than the accommodation space of the guide unit 111 and the drug injection unit 112 to communicate with each other, and is disposed at a point perpendicular to the central axis of the guide unit 111 and the drug injection unit 112. The air discharge unit 115 is supplied with the internal air or drugs from the drug injection unit 112 and discharges them to the lower part thereof.

In particular, the air discharge unit 115 is allowed to have the air discharge hole 121a of the guide needle 121 to be located thereon so that air is discharged to the lower end of the guide needle 121. Additionally, the air discharge unit 115 is formed such that the space expands widely, and thus, it is provided such that so that the air discharge hole 121a of the guide needle 121 is located in the lateral direction of the drug injection unit 112 even if the air discharge hole 121a is not precisely positioned. Such an air discharge unit 115 can completely remove air in the drug injection tube 230 and the injection needle 210 due to its shape and configuration.

The catheter insertion unit 116 is one which provides a space in which the catheter 150 can be inserted into a lower part of the body member 110, and is extended in a downward direction of the guide unit 111, and thereby an enlarged space for adhesion is formed to the outside of the lower surface. In particular, the space for adhesion of the catheter insertion unit 116 is the space where the adhesive A is applied after the catheter 150 is inserted, and it is formed so that the catheter 150 can be fixed to a catheter insertion unit 116. Additionally, a groove inclined downward is formed in the space for adhesion of the catheter insertion unit 116, and thus, it is possible to prevent an overflow to the outside when the adhesive A is applied.

Additionally, a UV curable adhesive that can be cured by ultraviolet rays can be used as the adhesive A, and in an embodiment, the adhesive A may include one or more reactive oligomers among acrylate-based, polyester-based, polyether-based, urethane-based, epoxy-based, and silicone-based reactive oligomers.

In this case, since the catheter 150 is not made of Teflon, but made of polyurethane, not only the catheter 150 is firmly adhered and fixed to the catheter insertion unit 116, but also the space of the catheter insertion unit 116 is completely sealed by the adhesive A, and thus there is an effect that air does not flow into the inside or the insulin solution present inside is not discharged to the outside, in the case where the adhesive A is applied to the adhesive space and cured by ultraviolet rays.

Additionally, not only the manufacturing cost is reduced because there is no need to use a separate fixing member, but also the conventional fixing member manufacturing process and coupling process are all reduced and the catheter 150 can be fixed simply by applying the adhesive A and curing it with ultraviolet rays, there are remarkable effects of reducing procedural steps and the costs involved therein.

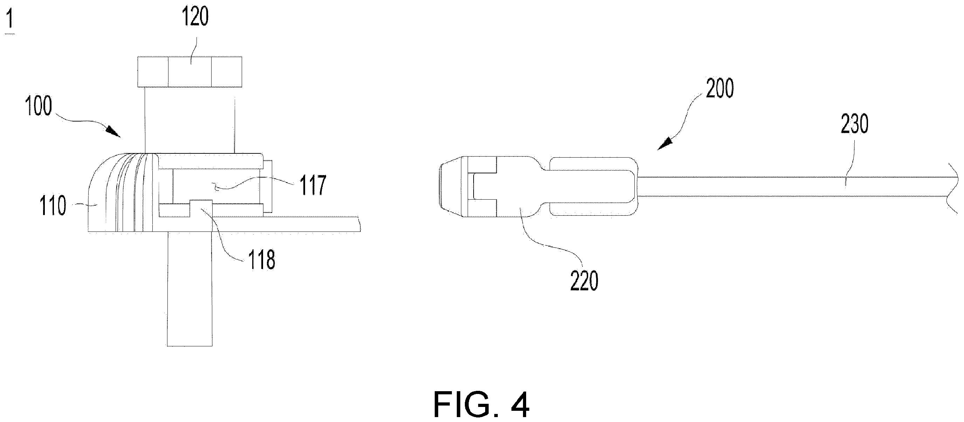

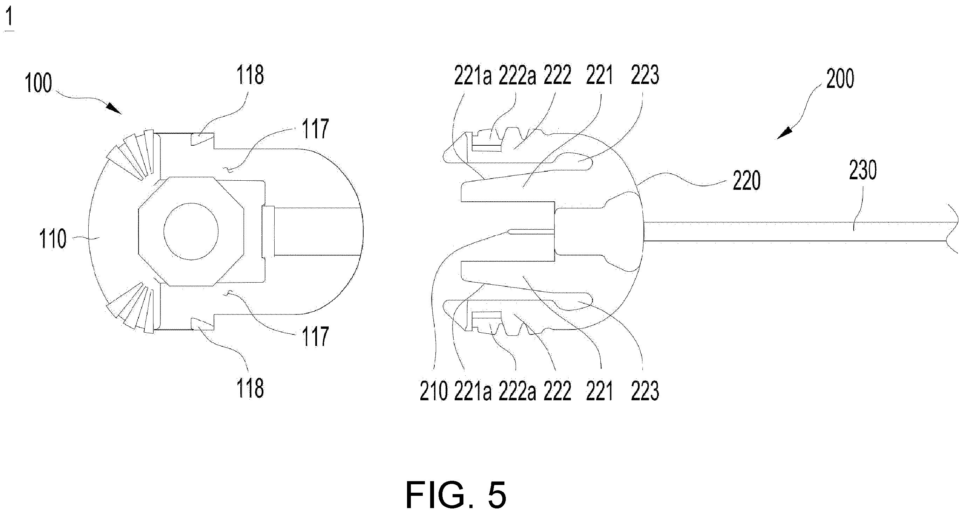

FIG. 4 is a front view showing a state before a catheter fixing unit and a drug infusion unit of the present disclosure are coupled together and FIG. 5 is a plan view showing a state before a catheter fixing unit and a drug infusion unit of the present disclosure are coupled together.

Referring to FIGS. 4 and 5, the insertion guide groove 117 is a constitution which guides the drug infusion unit 200 to be inserted into the correct position of the catheter fixing unit 100, and it is symmetrically formed so that a groove of a pre-set length is opened from the other side of the body member 110 to one side thereof. In particular, the insertion guide groove 117is formed so that the lower surface of the body member 110 remains to have the pre-set thickness, and the drug infusion unit 200 is formed to be inserted as if it were sliding along the insertion guide groove 117 at the bottom surface of the body member 110.

The locking protrusion 118 is a constitution in which the drug infusion unit 200 is inserted into the catheter fixing unit 100 and then locked to be fixed. It is symmetrically formed at the front and rear ends of the insertion guide groove 117 and it consists of a right-angled triangle, and the right-angle side is disposed on one side and a slope is disposed on the other side thereof. Such a locking protrusion 118 is retracted along the slope of a locking protrusion 118 as the drug infusion unit 200 moves to the catheter fixing unit 100 side by a right triangle, and then is opened again to be fixed at the slope end position of the locking protrusion 118, which is a state where it is fully inserted into the insertion guide groove 117.

As shown in FIGS. 1 and 3, the guided insertion member 120 is a constitution in which it is inserted into the catheter 150 through the guide unit 111 so that the catheter 150 can be inserted into the patient's body and removes the air from the inside through the air discharge hole 121a. Such a guided insertion member 120 may include a guide needle 121, a guide needle cap 122, and a protection cap 123.

The guide needle 121 is a constitution which helps the catheter 150 to be smoothly inserted into the patient's body, and it helps the catheter 150 to be inserted into the patient's body in a state where it penetrates the first sealing member 130 and is inserted into the inside of the catheter 150 of a flexible material. In particular, the guide needle 121, on which an air discharge hole 121a is formed in a middle point thereof, is disposed in the direction of the drug injection unit 112. The guide needle 121 allows the air in the drug injection tube 230 and the injection needle 210 to be introduced through the air discharge hole 121a while being transported to the air discharge unit 115 by operating a pump and removed by being discharged to the outside.

The guide needle cap 122 is formed so that a patient or user can directly insert it into the upper part of the body member 110. It can be formed in a size corresponding to the body member 110 so that the user can hold it with his/her hand and insert it into the body member 110 or easily remove it. Additionally, the guide needle cap 122 can be seated on the horizontal surface of the outer circumferential surface of the fusion groove 114 when inserted, so that the inserted state can be maintained in a stable state.

The protection cap 123 is a constitution which protects the patient or user from being pierced or injured by the guide needle 121. It is of a cylindrical shape and is disposed at the lower end of the body member 110 to be spaced apart from the outer peripheral surface of the catheter 150, and is coupled so that it can be mounted and unmounted so as to protect the catheter 150 and the guide needle 121 from the outside. In particular, the protection cap 123 can be easily fixed to the body member 110 because a groove in a shape corresponding to that of the protection cap 123 is formed on the lower surface of the body member 110.

The first and second sealing members 130 and 140 are constitutions which prevent leakage to the outside when a drug is injected, and they are inserted into the accommodation space of the guide unit 111 and the drug injection unit 112 and have the function of sealing the inside. Such first and second sealing members 130 and 140 are preferably formed to correspond to the shapes of the guide unit 111 and the drug injection unit 112. Since they are made of a carbon material, they can prevent leakage by being expanded by the elastic material properties of 130 and 140, even when they are removed after the guide needle 121or the injection needle 210 is inserted. Additionally, the first and second sealing members 130 and 140 have a waterproof function that prevents foreign matters or water from entering the inside thereof.

The catheter 150 is a constitution which is inserted into the body by the guide needle 121 so that a drug can be administered to the patient. Since the catheter 150 consists of polyurethane, it can move flexibly to prevent departure even if the drug infusion set 1 is shaken or shocked by the patient's movement. Such a catheter 150 is inserted and fixed through the catheter insertion unit 116 to surround the lower outer circumferential surface of the guide needle 121, and it is applied with an adhesive, and fixed by curing with UV ultraviolet rays.

Conventionally, the catheter 150 was made of the expensive Teflon material and thus could not be fixed using an adhesive. For this reason, the shape and assembly method had to be implemented in a complicated way so that the catheter 150 could be fixed to the body member 110. In the present disclosure, the catheter 150 is made of polyurethane material and thus can be easily fixed using an adhesive and the shape and assembly structure of the body member 110 can be simply changed.

Additionally, since the catheter 150 is made of a polyurethane material instead of a Teflon material, the manufacturing cost is reduced, and the constitution is simplified such that the catheter can be fixed by an adhesive cured by ultraviolet rays, and thus, the time required for assembly is shortened and the procedural steps are reduced thus shortening the total working time, thereby reducing the manufacturing cost and the purchase cost, resulting in lower sales cost.

Referring to FIGS. 4 and 5 again, the drug infusion unit 200 is one which is formed so that the drug can be supplied to the catheter fixing unit 100, and it is connected to the drug supply pump so as to be mounted/unmounted to the catheter fixing unit 100. Such a drug infusion unit 200 may include the composition of an injection needle 210, a drug injection member 220, and a drug injection tube 230.

The injection needle 210 is formed so that a drug can be supplied to the drug injection unit 112 of the body member 110. The drug is injected by the operation of a pump in a state where the drug penetrates the second sealing member 140 and is inserted into the injection needle location groove 112b, and is supplied to the drug introduction groove 112c. In particular, the injection needle 210 is positioned on the horizontal line with the air discharge hole 121a of the guide needle 121, and the air inside the an injection needle 210 is first discharged to the outside before injecting the drug into the patient's body.

The drug injection member 220 is a constitution which is coupled with the body member 110 so that the injection needle 210 can be inserted into the correct position so as to supply a drug smoothly. The drug injection member 220 consists of a structure that can be separated and coupled with the catheter fixing unit 100, and is provided to be installed to supply a drug only when the drug is injected. Such a drug injection member 220 may include a configuration of a fixed wing unit 221, a modified wing unit 222, and an elasticity guide groove 223.

The fixed wing unit 221 is a constitution which is inserted and coupled along the insertion guide groove 117 of the body member 110. The fixed wing unit 221 is formed to be spaced from both sides of the injection needle 210 and has a slope of 221a where one side of the outer surface is inclined downward. Such a slope 221a of the fixed wing unit 221 is formed so that the modified wing unit 222 can be retracted inward and thereby can extend the range of motion of the modified wing unit 222. Additionally, the fixed wing unit 221 helps to smoothly perform the removal and installation of the drug infusion unit 200 from the catheter fixing unit 100.

The modified wing unit 222 is a constitution in which it is locked to be fixed by the locking protrusion 118 of the body member 110 by elastic deformation or separated, and it is formed to be spaced apart from both sides of the fixed wing unit 221 to form an outward curved shape repeatedly. Due to the curved shape of a modified wing unit 222, the modified wing unit 222 can be retracted inward or elastically deformed to return to its original state. Therefore, the elasticity improves as the curved shape is repeated on the outer surface of the modified wing unit 222.

Additionally, a slope corresponding to the locking protrusion 118 is formed at one end of the modified wing unit 222, one end of the modified wing unit 222 moves as if it were sliding inward on the slope of the locking protrusion 118 as the drug infusion unit 200 is inserted into the catheter fixing unit 100, the modified wing unit 222 is retracted inward. Additionally, the internal surface of the modified wing unit 222 is retracted to the maximum extent while touching the slope of the fixed wing unit 221, and it is restored to its original state after being inserted to the end of a slope of locking protrusion 118.

In particular, a wing fixing groove 222a into which the locking protrusion 118 is inserted is formed on the upper outer surface of the modified wing unit 222. Therefore, when the drug infusion unit 200and the catheter fixing unit 100 are coupled, while being inserted into the wing fixing groove 222a, it is locked and restored to its original state after the modified wing unit 222 is retracted along the locking protrusion 118. Additionally, when the drug infusion unit 200 and the catheter fixing unit 100 are separated, the locking protrusion 118 is removed from the wing fixing groove 222a and separated, as it is pulled to the other side in a state where the outer surface of the modified wing unit 222 is retracted under pressure.

An elasticity guide groove 223 is a constitution which improves the range of elastic deformation of the modified wing unit 222, and it is formed at the connecting point on the other side between the fixed wing unit 221 and the modified wing unit 222, and is formed in the shape of a groove extending outward. In particular, the elasticity guide groove 223 is provided such that there is a groove formed to be deeper than the internal surface of the modified wing unit 222, to thereby prevent the other end of the modified wing unit 222 from being broken by the elastic deformation of the front end. Such an elasticity guide groove 223 can prevent damage from repeated elastic deformation of the modified wing unit 222 and helps it to move flexibly.

The drug injection tube 230 is formed in the shape of a long hose, is connected to the other end of the injection needle 210, and is provided through the drug injection member 220. Additionally, the drug injection tube 230 is provided so that drugs can be supplied to patients by supplying drugs to the injection needle 210 by a pump.

Thus far, the preferred embodiments of the present disclosure have been described. According to the drug infusion set according to embodiments of the present disclosure consisting of the above constitutions, the drug infusion set has the following effects.

Since the drug infusion set consists of a simple structure in which the catheter fixing unit and the drug infusion unit can be mounted/unmounted, and thus, it is easy for anyone to use by removing the guide needle and injecting the drug into the body through the catheter in a state where the catheter and the guide needle of the catheter fixing unit are inserted into the human body, and drugs can be administered periodically without multiple injections into the body.

Additionally, as the catheter is prepared using a polyurethane material instead of a Teflon material, the cost is reduced, and the constitution is simplified to fix the catheter by curing the adhesive with ultraviolet rays. As a result, the assembly time is saved and the procedural steps are reduced, and the total working time is shortened, thereby reducing the manufacturing cost and purchase cost, and lowering the sales cost.

Additionally, since the body member is integrally molded, it does not require elaborate work, and the first and second sealing members are inserted into an accommodation space of the body member and completely seal the interior, and thus, it is possible to seal them so as to prevent leakage when drugs are injected, and accidents caused by drug leakage can be prevented by stably supplying drugs through the guide needle and the injection needle.

Additionally, the air discharge hole of the guide needle is arranged to be positioned in the air discharge unit inside of the body member so that the air is discharged up to the tip of the guide needle. Since the space of an air discharge unit is formed to expand widely, the air in the drug injection tube and the injection needle can be completely removed even if the air discharge hole of the guide needle is not precisely positioned.

## Claims

1. A drug infusion set (1), in which a catheter fixing unit (100) and a drug infusion unit (200) are mounted/unmounted, wherein the catheter fixing unit (100) comprising:
a body member (110), which comprises:
a guide unit (111), in which a receiving space of a pre-set shape is formed in a vertical downward direction from an upper surface,
a drug injection unit (112), in which an accommodation space of a pre-set shape is formed in a horizontal direction from other side to communicate with the guide unit (111), and into which the drug infusion unit (200) is inserted; and
a catheter insertion unit (116), which extends in a downward direction of the guide unit (111) to form an adhesive space that extends to an outside of a lower surface;
a guided insertion member (120), which is provided with a guide needle (121), which is inserted into the guide unit (111), disposed to be exposed to the outside through the catheter insertion unit (116) in a state where an air discharge hole (121a) is formed at a point where the guide unit (111) is communicated with the drug injection unit (112), and being pierced through a first sealing member (130);
a first sealing member (130) and a second sealing member (140), which are inserted into the receiving space of the guide unit (111) and the accommodation space of the drug injection unit (112), so as to seal an inside thereof;
a catheter (150), which is inserted into the catheter insertion unit (116) and is formed such that the guide needle (121) is allowed to be separated while surrounding a lower outer circumferential surface of the guide needle (121); and
a flow path, in which the catheter (150) consists of polyurethane, and an adhesive, which is injected from the adhesive space of the catheter insertion unit (116), is cured by ultraviolet rays to fix the catheter (150), and a drug is moved inside the body member (110).

2. The drug infusion set (1) of claim 1, wherein the body member (110) comprises an air discharge unit (115), where a discharge space smaller than the receiving space of the guide unit (111) and the accommodation space of the drug injection unit (112) is formed to communicate, and is disposed at a point orthogonal to a central axis of the guide unit (111) and the drug injection unit (112), and
wherein the air discharge hole (121a) of the guided insertion member (120) is disposed in other direction of the air discharge unit (115), so as to discharge an internal air of the drug infusion unit (200).

3. The drug infusion set (1) of claim 1, wherein the body member (110) comprises:
a fusion protrusion unit (113), which is formed on a top of the guide unit (111) and on a side of the drug injection unit (112), and is fused to upper surfaces of the first sealing member (130) and the second sealing member (140) as a projection with a pre-set thickness protrudes along an outer circumferential surface; and
a fusion groove (114), which is formed at a set depth along the outer circumferential surface of the fusion protrusion unit (113) on the upper surface,
wherein in the fusion protrusion unit (113), the fusion groove (114) guides an movement of a thermal fusion device, so that the fusion protrusion unit (113) is fused to the inside by a thermal welding device and is fused to a surface exposed to the outside of the first sealing member (130) and the second sealing member (140), so as to fix the first sealing member (130) and the second sealing member (140).

4. The drug infusion set (1) of claim 1, wherein the guided insertion member () comprises: 120
a guide needle cap (122), which is coupled to a top of the guide needle (121) to separate the guide needle (121) from the body member (110); and
a protection cap (123), which is coupled to a bottom of the body member (110) to be separated from an outer circumferential surface of the catheter (150), and is mounted and removed, so as to protect the catheter (150) and the guide needle (121) from the outside.

5. The drug infusion set (1) of claim 1,
wherein the body member (110) comprises:
an insertion guide groove (117), which is symmetrically formed so that a groove with a pre-set length is opened from a front to a rear from other side to one side; and
a locking protrusion (118), which is symmetrically formed at front and rear ends of the insertion guide groove (117), consists of a right-angled triangle such that a right-angled surface is disposed on one side and a slope (221a) is placed on other side; and
wherein the drug infusion unit (200) comprises:
an injection needle (210), in which one side is inserted into the drug injection unit (112) and through which the drug is injected in a state where the injection needle (210) is penetrated through the second sealing member (140);
a drug injection member (220), which comprises:
a fixed wing unit (221), in which one side is inserted into the insertion guide groove (117), which is formed to be spaced apart to both sides of the injection needle (210) and has a slope where an outer surface is inclined downward to one side thereof;
a modified wing unit (222), in which one side is inserted into inside of the locking protrusion (118) to be fixed, and is formed to be spaced apart to both sides of the fixed wing unit (221) and is bent outward; and
an elasticity guide groove (223), which is formed at a connecting point on other side of the fixed wing unit (221) and the modified wing unit (222) and is formed to extend outwardly; and
a drug injection tube (230), in which the injection needle (210) is connected to other end, which is provided through the drug injection member (220), and which supplies the drug to the injection needle (210) by a pump.

## Amended claims

### Amended claims in accordance with Rule 137(2) EPC.

1. A drug infusion set (1), wherein a catheter fixing unit (100) and a drug infusion unit (200) are mounted/unmounted in the drug infusion set (1), wherein the catheter fixing unit (100) comprises:
a body member (110), which comprises:
a guide unit (111), wherein a receiving space of a pre-set shape is formed in a vertical downward direction from an upper surface,
a drug injection unit (112), wherein an accommodation space of a pre-set shape is formed in a horizontal direction from other side to communicate with the guide unit (111), and the drug infusion unit (200) is inserted into the drug injection unit (112); and
a catheter insertion unit (116), which extends in a downward direction of the guide unit (111) to form an adhesive space that extends to an outside of a lower surface;
a guided insertion member (120), which is provided with a guide needle (121), wherein the guide needle (121) is inserted into the guide unit (111) and disposed to be exposed to the outside through the catheter insertion unit (116) in a state where an air discharge hole (121a) is formed when the guide unit (111) is communicated with the drug injection unit (112), and the guide needle (121) pierces through a first sealing member (130);
the first sealing member (130) and a second sealing member (140), which are inserted into the receiving space of the guide unit (111) and the accommodation space of the drug injection unit (112), so as to seal an inside thereof;
a catheter (150), which is inserted into the catheter insertion unit (116), wherein the guide needle (121) is separated when the catheter (150) surrounds a lower outer circumferential surface of the guide needle (121); and
a flow path,
wherein the drug infusion set (1) is **characterized in that** the catheter (150) consists of polyurethane, and an adhesive injected from the adhesive space of the catheter insertion unit (116) is cured by ultraviolet rays to fix the catheter (150), and a drug is moved inside the body member (110).

2. The drug infusion set (1) of claim 1, wherein the body member (110) comprises an air discharge unit (115), a discharge space smaller than the receiving space of the guide unit (111) and the accommodation space of the drug injection unit (112) is formed to communicate, and is disposed at a point orthogonal to a central axis of the guide unit (111) and the drug injection unit (112),
wherein the air discharge hole (121a) of the guided insertion member (120) is disposed in other direction of the air discharge unit (115), so as to discharge an internal air of the drug infusion unit (200).

3. The drug infusion set (1) of claim 1, wherein the body member (110) comprises:
a fusion protrusion unit (113), which is formed on a top of the guide unit (111) and on a side of the drug injection unit (112), and is fused to upper surfaces of the first sealing member (130) and the second sealing member (140) as a projection with a pre-set thickness protrudes along an outer circumferential surface; and
a fusion groove (114), which is formed at a set depth along the outer circumferential surface of the fusion protrusion unit (113) on the upper surface,
wherein in the fusion protrusion unit (113), the fusion groove (114) guides a movement of a thermal fusion device, so that the fusion protrusion unit (113) is fused to the inside by a thermal welding device and is fused to a surface exposed to the outside of the first sealing member (130) and the second sealing member (140), so as to fix the first sealing member (130) and the second sealing member (140).

4. The drug infusion set (1) of claim 1, wherein the guided insertion member (120) comprises:
a guide needle cap (122), which is coupled to a top of the guide needle (121) to separate the guide needle (121) from the body member (110); and
a protection cap (123), which is coupled to a bottom of the body member (110) to be separated from an outer circumferential surface of the catheter (150), and is mounted and removed, so as to protect the catheter (150) and the guide needle (121) from the outside.

5. The drug infusion set (1) of claim 1,
wherein the body member (110) comprises:
an insertion guide groove (117), which is symmetrically formed so that a groove with a pre-set length is opened from a front to a rear from other side to one side; and
a locking protrusion (118), which is symmetrically formed at front and rear ends of the insertion guide groove (117), consists of a right-angled triangle such that a right-angled surface is disposed on one side and a slope (221a) is placed on other side; and
wherein the drug infusion unit (200) comprises:
an injection needle (210), wherein one side is inserted into the drug injection unit (112) and the drug is injected through the injection needle (210) in a state where the injection needle (210) penetrates through the second sealing member (140);
a drug injection member (220), which comprises:
a fixed wing unit (221), wherein one side is inserted into the insertion guide groove (117), the fixed wing unit (221) is spaced apart from both sides of the injection needle (210) and has a slope where an outer surface is inclined downward to one side thereof;
a modified wing unit (222), wherein one side is inserted into inside of the locking protrusion (118) to be fixed, and is formed to be spaced apart from both sides of the fixed wing unit (221) and is bent outward; and
an elasticity guide groove (223), which is formed at a connecting point on other side of the fixed wing unit (221) and the modified wing unit (222) and is formed to extend outwardly; and
a drug injection tube (230), wherein the injection needle (210) is connected to other end, the drug injection tube (230) is provided through the drug injection member (220), which supplies the drug to the injection needle (210) by a pump.
